# EUROPEAN PATENT APPLICATION

(11) **EP 2 103 246 A2**
(43) Date of publication of application: **23.09.2009**
(21) Application number: 09003688.0
(22) Date of filing: 13.03.2009
(51) Int. Cl.: A61B 1/00, A61B 1/005

(54) **Endoscope**

(30) Priority: 17.03.2008 JP 2008067762; 19.03.2008 JP 2008071804
(71) Applicant: FUJIFILM Corporation, Minato-ku Tokyo (JP)
(72) Inventor: Matsunaga, Jun, Ashigara-kami-gun Kanagawa (JP); Ueda, Yoshihiro, Ashigara-kami-gun Kanagawa (JP)
(74) Representative: Klunker . Schmitt-Nilson . Hirsch

(57) **Abstract**

An endoscope (10) comprises: an insertion tube (12) to be inserted into a body cavity, the insertion tube including a hard distal end portion (20), a curving portion (22) continuous with the hard distal end portion, and a flexible portion (24) continuous with the curving portion; a contained part (42,50) of the insertion tube which is accommodated at least inside the curving portion; and at least one movement regulating member (70) which extends inside the curving portion and adapted to regulate at least radial movement of the contained part, one end portion (70b) of the position regulating member passing through a rear connecting portion (72) connecting the curving portion and the flexible portion to extend to the flexible portion side, a section of a portion of the movement regulating member inserted into the flexible portion from the rear connecting portion being smaller than a section of a portion of the movement regulating member within the curving portion (70a).

## Description

The entire contents of literatures cited in this specification are incorporated herein by reference.

### BACKGROUND OF THE INVENTION

The present invention relates to an endoscope including an insertion tube which is inserted into a body cavity and includes a hard distal end portion, a flexible portion, and a curving portion arranged between the hard distal end portion and the flexible portion.

Generally speaking, an endoscope includes a flexible insertion tube to be inserted into a body cavity, and an operating portion which is used to operate the insertion tube to control an advancing direction thereof and to perform image control. Further, the insertion tube includes a hard distal end portion, a curving portion formed by rotatably connecting rings together and operated by the operating portion so as to be curved, and a flexible portion.

Further, as contained parts of the insertion tube, there are inserted, into the curving portion and the flexible portion of the insertion tube, several cables and tubes such as a cable like a signal line for transferring images and pictures taken by an image pickup device (CCD sensor) fixed to the hard distal end portion, and light guides for guiding into the hard distal end portion light for illuminating the interior of the body cavity. Further, also inserted into the insertion tube are tubes forming a forceps channel for inserting a forceps for conducting treatment by a treatment device while observing the object of inspection, an air-supply/water-supply channel for blowing air and water against the object of inspection in order to secure the observation visual field for the object of inspection, and a suction channel for, conversely, sucking water and air from the object of inspection.

Those contained parts of the insertion tube, consisting of cables and tubes, are curved inside the curving portion when the curving portion of the insertion tube is curved. It should be noted, however, that, at the time of curving, there is a difference in curving arc length between the contained parts on the outer side and the contained parts on the inner side, and hence the contained parts make not only an axial movement but also a radial movement. Further, by repeating this curving, the radial movement amount of some particular cables and tubes increases. Then, they intrude between the other cables and tubes to disorder the arrangement and cause mutual twisting and entanglement of the contained parts, thereby damaging the covering of the contained parts or making it impossible for the curving portion to be smoothly curved or turned back.

In order to solve the above-mentioned problem in the prior art, each of JP 3181707 B and JP 2001-137178 A discloses a technology according to which there is axially inserted between the contained parts a regulating member for regulating the radial movement of the contained parts, thereby allowing the cables and tubes to move solely in the axial direction.

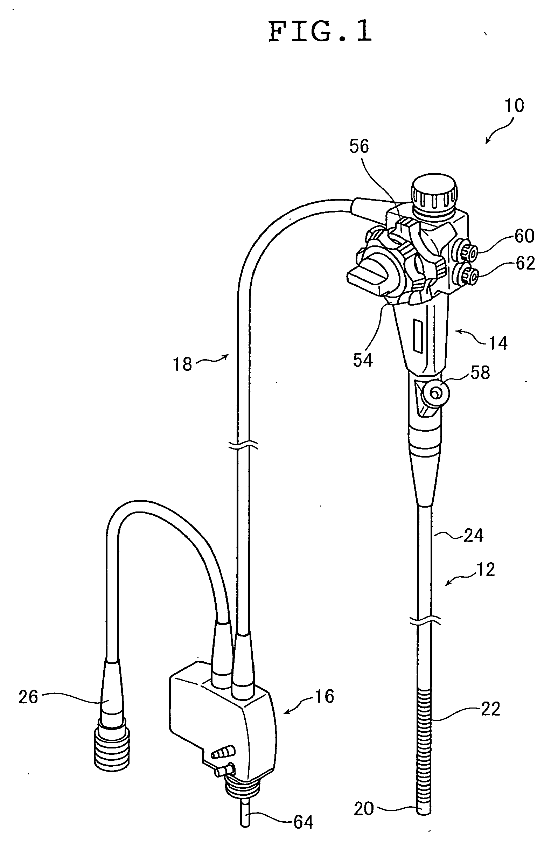

JP 3181707 B discloses an endoscope having a contained-parts movement regulating member that is tapered so as to be changed in width in the axial direction. This movement regulatingmember is formed in a substantially pyramid-like configuration at an appropriate position in a curving tube portion (See paragraph 0024 of the publication). Further, FIG. 1 of the publication illustrates how the movement regulating member, which is substantially of the same length as a distal end portion, is inserted into the curving tube portion from the rear end of the distal end portion.

This movement regulating member is arranged in order not to move the contained parts in directions other than the axial direction. Regarding the configuration and sectional rigidity of the movement regulatingmember, in order not to move the contained parts in directions other than the axial direction, there is made a disclosure or suggestion to the effect that the movement regulating member has a rigidity that is approximately of the same level as or higher than that of the contained parts.

JP 2001-137178 A discloses an endoscope in which a movement regulating member formed by a metal hollow coil is arranged in a gap portion generated between the contained parts within the curving portion. Regarding this movement regulating member, there is a description to the effect that it extends over the entire length of the curving portion into the flexible tube (See paragraph 0014 of the publication), and that it is only necessary for the movement regulating member to be arranged at least in the curving portion (See paragraph 0020 of the same). However, there is no description regarding the terminal end portion of the movement regulating member.

The movement regulating member as disclosed in JP 2001-137178 A is a coil pipe formed by spirally close-winding a stainless steel wire material of a circular sectional configuration in a fixed diameter or a spiral pipe formed by spirally winding a stainless steel strip material in a fixed diameter. In both cases, it exhibits a very high rigidity with respect to sectional deformation of the movement regulating member.

According to the technologies as proposed in JP 3181707 B and JP 2001-137178 A, radial movement is suppressed through interference between the contained parts and the movement regulating member, and only axial movement is allowed.

However, when such a movement regulating member is inserted into the insertion tube, the filling ratio of the interior of the insertion tube increases, and hence the resistance offered to the axial movement of the contained parts increases. In particular, from the structural point of view, the filling ratio tends to be rather high at the connecting portion (rear connecting portion) between the curving portion and the flexible portion. When the movement regulating member is inserted into this connecting portion, the resistance offered to the axial movement is markedly high. That is, in this connecting portion, where the curving portion and the flexible portion are connected together, the wall thickness of the insertion tube is relatively large, which means its inner diameter is small, with the result that the filling ratio at the connecting portion between the curving portion and the flexible portion is rather high. Thus, an increase in the resistance offered to the axial movement of the contained parts at the connecting portion is inevitable.

It might be possible to adopt a construction in which the axial length of the movement regulating member is adjusted such that it does not reach the connecting portion between the curving portion and the flexible tube, where the filling ratio is particularly high. In this case, however, an end portion of the movement regulating member would get caught in a gap generated between the rings during the curving motion, resulting in various problems: for example, the end portion would stick out through the gap between the rings; or the movement regulating member would undergo buckling.

That is, if, as illustrated in a drawing (FIG. 1) of JP 3181707 B, in order to avoid an increase in the resistance offered to the axial movement of the contained parts in the connecting portion, the movement regulating member, the length of which is reduced to substantially the same length as the distal end portion, is inserted into the curving portion from the rear end of the distal end portion, the movement regulating member does not reach the position where the radial movement of the contained parts is maximum, that is, the minimum radius-of-curvature position where the radius of curvature of the curving portion is minimum, with the result that the movement regulation is not effectively performed on the contained parts at the position where the radial movement of the contained parts is maximum.

Further, if the end portion of the movement regulating member is extended to the position where the radial movement of the contained parts is maximum (i.e., minimum radius-of-curvature position where the radius of curvature of the curving portion is minimum), when the end portion of the movement regulating member exists within the curving portion, that end portion of the movement regulating member is likely to be held or caught in a gap between the rings forming the curving portion, and hence the rings do not move smoothly due to the obstruction by the end portion of the movement regulating member. In extreme cases, the end portion of the movement regulating member sticks out through the gap between the rings, or the movement regulating member is forcibly bent to undergo buckling.

If, in order to avoid such a problem, the movement regulating member is extended to the flexible portion, the filling ratio in the rear connecting portion increases as stated above, resulting in an increase in the resistance offered to the axial movement of the contained parts.

Further, even if, as disclosed in JP 3181707 B and JP 2001-137178 A, the movement regulating member is arranged inside the curving portion of the insertion tube, due to the difference in curving arc length between the contained parts on the outer side and those on the inner side, the contained parts move not only in the axial direction but also from the center toward the outer periphery, that is, radially, when the curving portion of the insertion tube is curved. Thus, in the actual curving motion of the curving portion of the insertion tube, there is exerted a force causing the contained parts to move toward the outer periphery, and hence the contained parts are pressurized by the movement regulating member to thereby suffer damage.

Further, it should be noted that, in order to attain the requisite functions, the contained parts are formed of materials appropriately selected, and each of them exhibits rigidity, etc. in correspondence with the material. For example, the contained parts such as the forceps channel and the air-supply/water-supply channel are hollow. However, it is necessary for the forceps channel to allow the forceps to freely pass therethrough, and it is necessary for the air-supply/water-supply channel to be capable of reliably passing water and air therethrough, and hence those contained parts exhibit a rigidity high enough to prevent them from being crushed even when the curving portion of the insertion tube is curved. However, though the contained parts can be curved, the contained parts, such as an image pickup data cable (harness) and a light guide (fiber bundle), are themselves covered with soft covering in order to secure the function of the contained-parts and to prevent damage thereof. Thus, inside the curving portion of the insertion tube, there exist together, as the contained parts, soft members covered with soft covering such as a data cable and a light guide, and members such as a forceps channel and an air-supply/water-supply channel which, though hollow, are hard to some extent, that is, which are endowed with some degree of rigidity and are hard to crush.

Here, even if the movement regulating member is arranged inside the curving portion of the insertion tube, when, as in the case of the conventional technologies disclosed in JP 3181707 B and JP 2001-137178 A, the rigidity of the movement regulating member is higher than that of the contained parts, for example, as in the case of the metal coil, the contained parts are pressed hard by the movement regulating member when the curving portion of the insertion tube is curved, with the result that they suffer damage.

Further, even when the movement regulating member is a bar-like or tubular member having elasticity, as disclosed in JP 3181707 B and JP 2001-137178 A, if its diameter is small and its rigidity is higher than that of the contained parts, the contained parts are pressed hard by the movement regulating member when the curving portion of the insertion tube is curved, with the result that they suffer damage.

When such a hard movement regulating member having a predetermined level of rigidity is thus held in contact with the contained parts formed of soft members, the hard movement regulating member is pressed hard against the soft members to crush the same when the curving portion is curved, and hence the soft members suffer damage such as deformation. Further, when the movement regulating member is strongly brought into contact with the soft members whose surface is soft, the soft members suffer damage such as a surface flaw of the covering thereof.

A wire guide into which a wire for curving the curving portion is inserted and held therein, is fixed to the inner surface of the curving portion and of an inwardly convex configuration. Further, the wire guide is formed of a hard material of very high rigidity, and hence, when, as in the case of the endoscopes disclosed in JP 3181707 B and JP 2001-137178 A, the contained parts that are soft members are held in contact with the wire guide, the contained parts, in particular, those formed of soft members are pressed hard by the hard wire guide when the curving portion is curved, with the result that the contained parts suffer damage.

### SUMMARY OF THE INVENTION

It is a first object of the present invention to solve the above-mentioned problems in the prior art and to provide an endoscope including a movement regulating member which prevents an end portion of the movement regulating member for suppressing radial movement of the contained parts in the curving portion from getting caught by rings of the curving portion to maintain slidability of the contained parts.

It is a second object of the present invention to solve the above-mentioned problems in the prior art and to provide an endoscope in which, inside the curving portion of the insertion tube, it is possible to prevent or mitigate damage of the contained parts that would be caused by the pressure on the contained parts from the movement regulating member.

An endoscope according to a first aspect of the present invention comprises: an insertion tube to be inserted into a body cavity, the insertion tube including a hard distal end portion, a curving portion continuous with the hard distal end portion, and a flexible portion continuous with the curving portion; a contained part of the insertion tube which is accommodated at least inside the curving portion; and at least one movement regulating member which extends inside the curving portion and adapted to regulate at least radial movement of the contained part, one end portion of the position regulating member passing through a rear connecting portion connecting the curving portion and the flexible portion to extend to the flexible portion side, a section of a portion of the movement regulating member inserted into the flexible portion from the rear connecting portion being smaller than a section of a portion of the movement regulating member within the curving portion.

An endoscope according to a second aspect of the present invention comprises: an insertion tube having a hard distal end portion and a flexible portion respectively on both sides of a curving portion that freely curves; a plurality of contained parts arranged inside the insertion tube; and at least one movement regulating member regulating radial movements of the contained parts, the movement regulating member being formed by a resin tube whose sectional configuration is capable of elastic deformation with respect to the adjacent contained parts.

An endoscope according to a third aspect of the present invention comprises: an.insertion tube having a hard distal end portion and a flexible portion respectively on both sides of a curving portion that freely curves, the insertion tube including a plurality of wire guides which are fixed to an inner surface thereof and which allow insertion of wires for curving the curving portion; a plurality of contained parts including a forceps channel arranged inside the insertion tube; and at least one movement regulating member regulating radial movements of the contained parts, the movement regulating member being arranged at a position in close proximity to outer peripheral portions of the wire guides situated to be opposed to an arrangement position of the forceps channel inside the curving portion, and being provided between the contained parts adjacent to the movement regulating member and the outer peripheral portions of the wire guides so that the adjacent contained parts may not be brought into direct contact with the outer peripheral portions of the wire guides.

### BRIEF DESCRIPTION OF THE DRAWINGS

In the accompanying drawings:
FIG. 1 is a perspective view illustrating general construction of an endoscope according to a first embodiment of the present invention;
FIG. 2 is a perspective view of a hard distal end portion used in the endoscope illustrated in FIG. 1;
FIG. 3 is a schematic sectional view of an insertion tube of the endoscope illustrated in FIG. 1;
FIG. 4 is a component view of another example of a movement regulating member used in another embodiment;
FIG. 5 is a sectional view illustrating how a contained part tends to make a radial movement when a curving portion of the insertion tube illustrated in FIG. 3 is curved;
FIG. 6 is a sectional view illustrating how another contained part tends to make a radial movement when the curving portion of the insertion tube illustrated in FIG. 3 is curved; and
FIG. 7 is a detailed sectional view illustrating construction of a curving portion of an insertion tube used in second embodiment.

### DETAILED DESCRIPTION OF THE PREFERRED EMBODIMENTS

Preferred embodiments of an endoscope of the present invention are described in detail below with reference to the accompanying drawings.

### FIRST EMBODIMENT:

First, an endoscope according to a first embodiment of the present invention is described with reference to FIGS. 1 through 6.

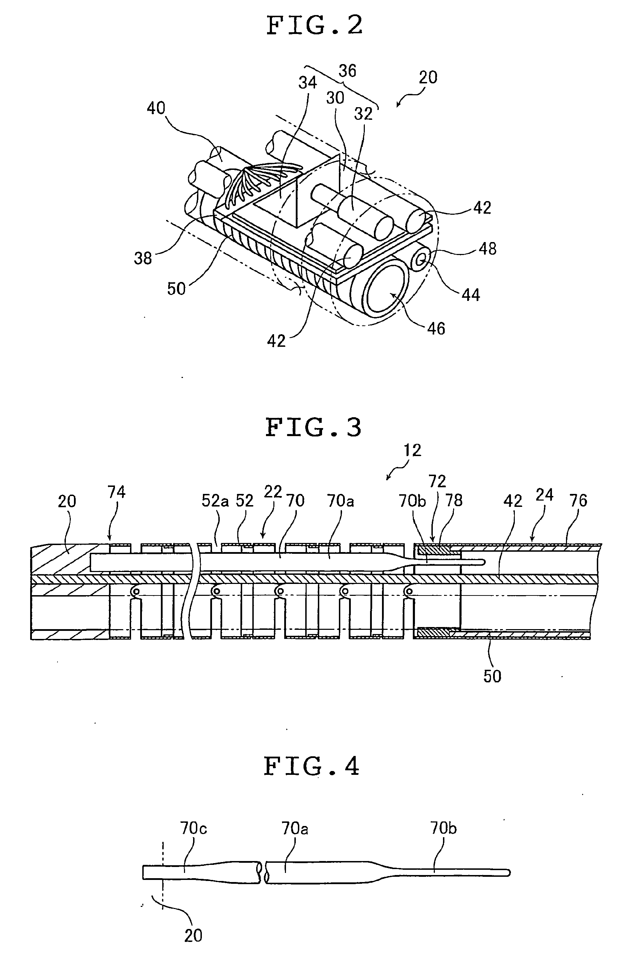

Here, FIG. 1 is a general perspective view of an endoscope 10 according to the first embodiment of the present invention, FIG. 2 is a perspective view of a hard distal end portion of the endoscope 10, and FIG. 3 is a schematic sectional view of an insertion tube of the endoscope 10.

As illustrated in FIG. 1, an endoscope 10 is a so-called electronic scope type endoscope which takes an image of an object of inspection such as an inside of a body cavity by using an image pickup device (described in detail below) such as a CCD sensor to directly observe the object of inspection as displayed on a display apparatus and to take a moving image or a still image of the object of inspection. As in the case of an ordinary endoscope, the endoscope 10 includes an insertion tube 12, an operating portion 14, a connector 16, a universal cord 18, etc, and at the distal end of the insertion tube 12, there is arranged a hard distal end portion 20 provided with an image pickup device (CCD camera) using a CCD sensor or the like.

In the endoscope 10, the insertion tube 12 is inserted into a portion to be inspected of the body cavity (alimentary canal, ear, nose, throat, etc.), and the object of inspection is photographed by the image pickup device such as the CCD sensor arranged in the hard distal end portion 20 to directly observe an image of the object, or to take a picture or a moving picture thereof. Further, a forceps (not shown) is inserted to perform gathering of tissue, etc. Further, the insertion tube 12 is a flexible, elongated member to be inserted into the object of inspection such as the body cavity, and formed of the hard distal end portion 20, a curving portion 22 and a flexible portion 24.

As illustrated in FIG. 2, inside the hard distal end portion 20, there is arranged a CCD sensor 30 for imaging the portion to be inspected. As an optical system for imaging the object of inspection by the CCD sensor 30, there are provided a lens 32 and a prism 34 for applying an image (light) entering the lens 32 to an imaging surface of the CCD sensor 30, with the whole forming an imaging portion 36 for imaging the object of inspection. An output signal from the CCD sensor 30 is output after undergoing a predetermined processing such as A/D conversion by a processing board 38.

Output signal lines for transmitting output signals from the processing board 38 are bundled into a single data cable 40. The data cable 40 is inserted into the curving portion 22 and the flexible portion 24 to be connected to a video connector 26 by way of the operating portion 14, the universal cord 18 and the connector 16, so that a moving image or a still image is displayed on a display apparatus (not shown) or a still image is printed out by a printer apparatus.

Further, two light guides 42 for projecting light for illuminating the object of inspection are arranged inside the hard distal end portion 20. Further, the hard distal end portion 20 has an air-supply/water-supply port 44 for supplying air, water, etc. to the object of inspection, a distal end side forceps port 46 for inserting a forceps for gathering tissue or the like up to the position of the object of inspection, etc. Further, connected to the air-supply/water-supply port 44 is an air-supply/water-supply channel 48 inserted into the insertion tube 12, and connected to the forceps port 46 is a forceps channel 50 inserted into the insertion tube 12.

Referring again to FIG. 1, the curving portion 22 is a region adapted to be curved vertically and horizontally (in four directions orthogonal to each other) through operation at the operating portion 14 in order to insert the hard distal end portion 20 to a desired position, and to photograph a target portion by the CCD sensor 30. In the endoscope 10 illustrated, as in the case of the curving portion of a conventional endoscope, the curving portion 22 is formed by a large number of circular rings 52 (See FIG. 3) that are connected together, with a wire (not shown) for curving the curving portion 22 being connected to the rings 52. As described below, the curving portion 22 is curved in an arbitrary direction through operation of an LR knob 54 and a UD knob 56 (See FIG. 1) of the operating portion 14.

The flexible portion 24 is a member connecting the hard distal end portion 20 and the curving portion 22 with the operating portion 14, and it is an elongated member having sufficient flexibility for insertion into the portion to be inspected. Further, in the curving portion 22 and the flexible portion 24, there are accommodated the contained parts such as the data cable 40, the light guides 42, the air-supply/water-supply channel 48, and the forceps channel 50, and inserted the wire for curving the curving portion 22, etc.

The operating portion 14 is a portion for performing various operations of the endoscope 10. As in the case of an ordinary endoscope, there are arranged in the operating portion 14, for example, apart from the LR knob 54 and the UD knob 56 mentioned above, an insertion side forceps port 58 which is an opening communicating with the forceps channel 50 and allowing insertion of the forceps, etc. as needed, a suction button 60 for effecting suction from the distal end side forceps port 46 of the hard distal end portion 20 via the forceps channel 50, an air-supply/water-supply button 62 for supplying air or water to the object of inspection from the air-supply/water-supply port 44 of the hard distal end portion 20 via the air-supply/water-supply channel 48. Apart from this, the operating portion 14 of the endoscope 10, which is an electronic scope, is provided with various switches for observing/taking images by the CCD sensor 30, such as a zoom switch, a still image taking switch, and a moving image taking switch.

Further, as described above, arranged on the operating portion 14 are the LR knob (left/right knob) 54 for curving the curving portion 22 to the left and to the right, and the UD knob (up/down knob) 56 for curving the curving portion 22 upwardly and downwardly. As in the various well-known endoscopes, also in the endoscope 10, by turning the LR knob 54 and the UD knob 56, the wire (not shown) connected to the curving portion 22 is pulled, whereby the interval between the rings 52 is varied, thereby curving the curving portion 22 vertically and horizontally. Through a combination of those motions, it is possible to effect curving arbitrarily in the vertical/horizontal directions.

The connector 16 is a member for connecting the endoscope 10 to a power source needed when using the same, a signal processing device for processing imaged signals, and a water supply unit, an air supply unit, a suction unit, etc., which are auxiliary units for facilitating image taking. Arranged on the connector 16 is an LG bar 64 for connecting the light guides 42 for illuminating the object of inspection to an illumination light source, a water supply connector (not shown) for connecting the endoscope 10 to the water supply unit, an air supply connector for connecting it to the air supply unit, a suction connector for connecting it to the suction unit, an S terminal for connecting an S cord when using an electric surgical knife, and the like.

The universal cord 18 is a member connecting the connector 16 and the operating portion 14 to each other, and has a power source cord for supplying power, the data cable for transmitting signals, and so on. Further, accommodated therein are the two light guides 42, the air supply pipe, the water supply pipe, the suction pipe, etc. connected to the air-supply/water-supply channel 48.

As stated above, the endoscope 10 is an electronic scope, and hence the connector 16 is further provided with the video connector 26 for connection, via the signal processing device, to a display apparatus such as a display, a television set or the like and an output apparatus such as a printer apparatus for printing out still images. Thus, an image taken by the CCD sensor 30 provided in the hard distal end portion 20 reaches the operating portion 14 via the data cable 40 of the insertion portion 12, and, from the operating portion 14, it is transmitted through the universal cord 18 by way of the connector 16 and connected to the signal processing device (not shown) via the video connector 26 to be connected to a display apparatus such as a television set and an output apparatus such as a printer apparatus for printing out still images.

FIG. 3 is a sectional view schematically illustrating the insertion tube 12 of the endoscope 10. As illustrated in the drawing, the insertion tube 12 includes the hard distal end portion 20, the flexible portion 24, and the curving portion 22 arranged between the hard distal end portion 20 and the flexible portion 24. Inside the insertion tube 12, there is arranged, together with the contained parts such as the light guides 42 and the forceps channel 50, at least one movement regulating member 70 regulating radial movement of the contained parts.

It is to be assumed that, when the curving portion 22 is curved, the radial movement amount of the contained parts is maximum at a minimum radius-of-curvature position, where the radius of curvature of the curving portion 22 is minimum. Thus, in the present invention, a large diameter portion 70a of the movement regulating member 70 having the maximum section is extended over the position where the radial movement of the contained parts are maximum, that is, over the minimum radius-of-curvature position where the radius of curvature of the curving portion 22 is minimum, making it possible to reliably effect, at the position where the radial movement of the contained parts are maximum, regulation on the radial movement of the contained parts.

Due to the construction of the curving portion 22, the minimum radius-of-curvature position, where the radius of curvature of the curving portion 22 is minimum, is not restricted to a single point but covers a fixed range, and hence it is desirable for the large diameter portion 70a to have the maximum section at least up to the center of the range of the minimum radius-of-curvature portion and, more preferably, substantially over the entire length of the minimum radius-of-curvature portion.

Thus, as illustrated in FIG. 3, in this embodiment, the large diameter portion 70a, which has the maximum section and regulates the position of the contained parts, extends up to a vicinity of a rear connection portion 72 connecting the curving portion 22 and the flexible portion 24 to each other, and is arranged so as to extend substantially over the entire length of the curving portion 22.

Further, in the portion where the positional regulation of the contained parts is not required, it is not necessary for the movement regulating member 70 to be formed as the large diameter portion 70a with maximum section, and hence, as in the case of one end portion (right-hand end portion in FIG. 3) of the movement regulating member 70, it is formed as a rear end side small diameter portion 70b with smaller diameter, which passes through the rear connecting portion 72 connecting the curving portion 22 and the flexible portion 24 to be situated on the flexible portion 24 side.

The other end portion (left-hand end portion in FIG. 3) of the movement regulating member 70 is inserted into the hard distal end portion 20 for fixation at a front connecting portion 74 where the hard distal end portion 20 and the curving portion 22 are connected together. Further, in this construction, even if' the movement regulating member 70 is pulled in the axial direction as a result of the curving of the curving portion 22, the movement regulating member 70 does not move in the axial direction. The manner of fixation of the other end portion (left-hand end portion in FIG. 3) of the movement regulating member 70 is not restricted to the one described above. Obviously, it is only necessary for the other end portion to be locked at an arbitrary position by an arbitrary locking device.

In the above embodiment illustrated in FIG. 3, the movement regulating member 70 inside the curving portion 22 has the same sectional configuration from the front connecting portion 74, where the curving portion 22 and the hard distal end portion 20 are connected together, to the minimum radius-of-curvature portion. However, as illustrated in FIG. 4, in the range requiring no positional regulation in the vicinity of the front connecting portion 74 connecting the movement regulating member 70 to the hard distal end portion 20, the movement regulating member 70 may be formed in a smaller diameter as a front-end side small diameter portion 70c. Due to this construction, it is possible to give some extra room to the front connecting portion 74, which is crowded with the image pickup portion 36, the mounting portion for the data cable 40, etc.

In this regard, the rear end side small diameter portion 70b and the front end side smaller diameter portion 70c may be of the same diameter, or, as illustrated in FIG. 4, of different diameters. Further, the lengths of the rear end side small diameter portion 70b and the front end side small diameter portion 70c may be determined as appropriate according to the length of the large diameter portion 70a, which is determined by the minimum radius-of-curvature position, where positional regulation for the contained parts is required.

As described above, the curving portion 22 is formed by rotatably connecting together the rings 52. As illustrated in FIG. 3, between the rings 52, there are provided gaps 52a for effecting curving. Thus, as described above, if an end portion of the movement regulating member 70 exists inside the curving portion 22, the end portion of the movement regulating member 70 may be held or caught between one of those gaps 52a forming the curving portion 22, and, in extreme cases, the end portion of the movement regulating member 70 may stick out of one of the gaps between the rings 52.

However, the movement regulating member 70 of the present invention is arranged such that one end portion (right-hand end portion in FIG. 3) thereof constitutes the rear end side small diameter portion 70b, which passes through the rear connecting portion 72 connecting the curving portion 22 and the flexible portion 24, and is situated on the flexible portion 24 side, and hence there is no fear of the end portion of the movement regulating member 70 being held or caught between the rings 52 forming the curving portion 22.

The rear connecting portion 72 is the portion connecting the curving portion 22 and the flexible portion 24 with each other, and it fixes together a close-contact spring 76 constituting the flexible member 24 and the rings 52 constituting the curving portion 22 by a close-contact spring fixing member 78. Thus, the wall thickness of the close-contact spring fixing member 78 becomes larger than that of the close contact spring 76 and the rings 52, with the result that a reduction in the inner diameter of the rear connecting member 72 is inevitable. In the movement regulating member 70 of the present invention, the section of the portion inserted into the flexibleportion 24 from the rear connecting portion 72 is that of the rear end side small diameter portion 70b, which is of small diameter, and hence it is smaller than the maximum section of the movement regulating member 70 in the curving portion 22, thus preventing an increase in the filling ratio and an increase in the resistance offered to the axial movement of the contained parts.

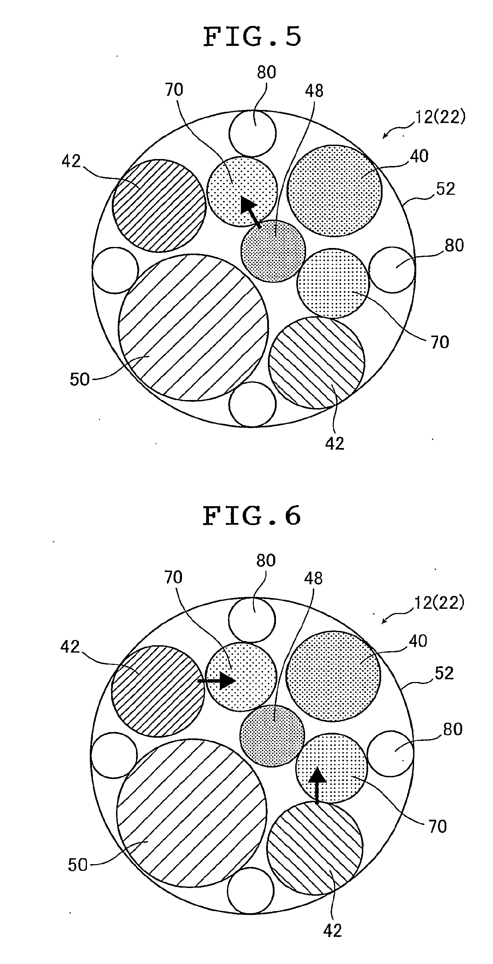

FIGS. 5 and 6 are sectional views illustrating how the different contained parts tend to make a radial movement when the curving portion 22 of the insertion tube 12 illustrated in FIG. 3 is curved, i.e., the drawings illustrate the way the contained parts make a radial movement when the curving portion 22 of the insertion tube 12 is curved. Passed through the insertion tube 12 are the data cable 40, the two light guides 42, the air-supply/water-supply channel 48, the forceps channel 50, and the two movement regulating members 70, and wire guides 80 are arranged on the upper and lower sides and the right-hand and left-hand sides of the rings 52, i.e. , four of them in total. Further, wires (not shown) for curving the curving portion 22 are inserted into the wire guides 80.

Next, to be illustrated are the results of observation of the behavior of the contained parts when the curving portion 22 of the insertion tube 12 having the contained parts is curved. Here, as the insertion tube 12, there is used one whose curving portion 22 has an outer diameter of 5. 9 mm and which has a length of 70 mm, and observed was the behavior of the movement regulating members 70 when the movement regulating members 70 are cut at a predetermined length from the front connecting portion 74 connecting the curving portion 22 and the hard distal end portion 20.

Further, the curving portion 22 was curved to the utmost, and the movement of the contained parts when the curving direction was varied while maintaining this curving state was observed. For example, in the insertion tube 12 (curving portion 22) illustrated in FIGS. 5 and 6, moving the contained parts from the upper left position to the upper right position means that the wire inserted into the left-hand wire guide 80 and the wire inserted into the upper wire guide 80 are pulled to the utmost to curve the curving portion 22 upwardly to the left to the utmost limit, and, in this state, the left-hand side wire is loosened and the right-hand side wire is pulled, with the upper side wire being kept pulled tight, whereby the curving direction is varied while maintaining the state in which the curving portion is curved to the utmost limit.

Table 1 illustrates the results of the above-mentioned observation.

**Table 1**

| Regulating member length | Disorder 1 | Disorder 2 |
|---|---|---|
| 30mm | × | × |
| 40mm | ○ | × |
| 50mm | ○ | ○ |
| 60mm | ○ | ○ |
| 70mm | ○ | ○ |

Here, disorder 1 indicates that, when, as illustrated in FIG. 5, the curving is performed in a direction from the upper left side to the upper right side, the air-supply/water-supply channel 48 moves in the direction of the arrow, and disorder 2 indicates that, when, as illustrated in FIG. 6, the curving is performed in a direction from the lower left side to the upper left side, the light guides 42 tend to move in the directions of the arrows. The evaluation was made visually to judge whether the radial movement of the contained parts is so large as to disorder the arrangement of the contained parts, and it is not whether the disorder is irreversible or not that matters here. Here, symbol "×" indicates large radial movement, and symbol "○" indicates small radial movement.

As is apparent from the above table, assuming that the entire length of the curving portion is 70 mm, when the length of the regulating member is substantially half the same, i.e., ranges from 30 mm to 40 mm, varying the curving direction while maintaining the state in which the curving portion is kept bent to the utmost results in a change in the judgment as to whether the arrangement of the contained parts has been disordered or not. This indicates that, even when the minimum radius-of-curvature position where the radius of curvature of the curving portion 22 is minimum is within a fixed range, it is desirable for the large diameter portion 70a to exhibit the maximum section at least up to the center of the range of the minimum radius-of-curvature position and, more preferably, substantially over the entire length of the range of the minimum radius-of-curvature position.

As described in detail above, the endoscope according to the first embodiment of the present invention is characterized in that, one end portion of the movement regulating member arranged in the insertion tube is passed through the rear connecting portion connecting the curving portion and the flexible portion of the insertion tube to be situated on the flexible portion side, and the section of the movement regulating member inserted into the flexible portion from the rear connecting portion is smaller than the maximum section of the movement regulating member inside the curving portion, and hence, even if the wall thickness of the insertion tube increases in the rear connecting portion and its inner diameter is diminished, the movement regulating member does not cause an increase in filling ratio, thus making it possible to provide an endoscope in which there is no increase in the resistance to the axial movement of the contained parts.

### SECOMD EMBODIMENT:

Next, an endoscope according to a second embodiment of the present invention is described.

FIGS. 1 through 3, which are referred to for the description of the endoscope of the first embodiment of the present invention, can also be referred to for the description of the endoscope of the second embodiment of the present invention, and hence a description of the drawings is omitted here.

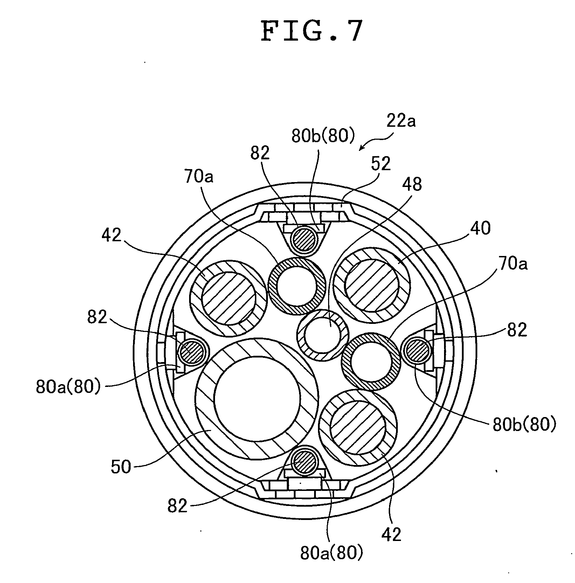

FIG. 7 is a cross-sectional view of a curving portion 22a of an insertion tube in the endoscope of the second embodiment of the present invention.

Inside the curving portion 22a, arranged is a plurality of contained parts such as the data cable 40, the light guides 42, the air-supply/water-supply channel 48 and the forceps channel 50. And movement regulating members 70a regulating radial movements of the contained parts are also arranged in the curving portion 22a together with the contained parts. The movement regulating members 70a are formed of resin tubes whose sectional configuration can undergo elastic deformation with respect to the inner surface of the curving portion 22a of the insertion tube or the contained parts.

There are no particular limitations regarding the resin tubes constituting the movement regulating members 70a as long as their sectional configuration is capable of elastic deformation with respect to the inner surface of the curving portion 22a of the insertion tube or the contained parts. It is desirable for them to consist of resin tubes softer than the covering of the adjacent contained parts such as the image taking data cable 40 and the light guides 42 adjacent to the movement regulating members 70a, that is, resin tubes of a material allowing sectional deformation more easily than the adjacent contained parts, and it is desirable for the outward rigidity of the resin tubes to be lower than the outward rigidity of the adjacent containedparts. Examples of the tubes include tubes of silicone rubber or soft vinyl chloride, and tubes formed of soft foam resin. In this case, it is desirable for the material forming the movement regulating members 70a to be one little subject to changes with passage of time and remaining soft and elastic.

As the curving portion 22 of the first embodiment illustrated in FIG. 3, the curving portion 22a is formed by connecting the rings 52 together. As illustrated in FIG. 7, arranged inside the rings 52 are wires 82 for curving the curving portion 22a, and the wire guides 80 into which the wires 82 are inserted to be supported therein. As is apparent from the drawing, the wire guides 80 are members fixed to the inner surface of the rings 52, protruding inwardly from the rings 52, and are required to be strong and rigid enough to allow insertion and supporting of the wires 82, and they are formed of a metal or a plastic of higher rigidity than the contained parts.

The forceps channel 50 is arranged between the left-hand side and the lower wire guides 80a (as seen in FIG. 7), which are adjacent to each other, of the four wire guides 80 into which the wires 82 for curving the curving portions 22a are inserted. In close proximity to two wire guides 80b, which are opposed to the two wire guides 80a, there are arranged the two movement regulating members 70a.

Further, the two light guides 42 are arranged on both sides of the forceps channel 50, the data cable 40 is arranged at a position opposed to the forceps channel 50, and the air-supply/water-supply channel 48 is arranged between the forceps channel 50 and the data cable 40. Here, the forceps channel 50 and the air-supply/water-supply channel 48 are hollow, and the data cable 40 and the two light guides 42 are solid.

As stated above, the contained parts such as the forceps channel 50 and the air-supply/water-supply channel 48 are hollow. However, the forceps channel 50 must allow a forceps (not shown) to be freely inserted thereinto, and the air-supply/water-supply channel 48 must reliably allow water and air to pass therethrough, and hence they are members which are rigid enough not to be crushed even when the curving portion 22a of the insertion tube is curved, that is, members which are hard to crush and which have a certain degree of hardness, that is, a certain degree of rigidity.

The contained parts such as the image taking data cable 40 and the two light guides 42 are soft members which can be curved and which are covered with soft covering in order to secure the respective functions and to prevent damage.

In this way, inside the curving portion 22a of the insertion tube, there exist together soft members covered with soft covering like the data cable 40 and the two light guides 42, and members which are hollow but have a certain degree of hardness, that is, a certain degree of rigidity, which makes them hard to crush like the forceps channel 50 and the air-supply/water-supply channel 48.

In the curving portion 22a of the insertion tube, the two movement regulating members 70a are arranged between the outer peripheral portions of the wire guides 80b and the air-supply/water-supply channel 48, and at positions where they are held between the data cable 40 and the light guides 42.

Further, as is apparent from FIG. 7, the size (diameter) of each movement regulating member 70a is larger than the gaps formed between the contained parts (data cable 40 and light guides 42) or between the inner surfaces (wire guides 80b) of the curving portion 22a and the contained part (air-supply/water-supply channel 48), and the' movement regulating members 70a are inserted into those gaps, with their sectional configuration being deformed.

In this embodiment, the wire guides 80 are provided at four positions in the circumferential direction of the curving portion 22a, and the forceps channel 50 is arranged between the left-hand side and the lower side wire guides 80a (as seen in FIG. 7) adjacent to each other, with the two movement regulating members 70a being arranged in close proximity to the remaining two wire guides 80b on the right-hand side and the upper side in FIG. 7.

As described in detail above, in the endoscope according to the second embodiment of the present invention, the movement regulating members are formed by resin tubes whose sectional configuration is capable of elastic deformation with respect to the adjacent contained parts, and hence, if there exist together, within the curving portion of the insertion tube of the endoscope, members hard to crush and soft members relatively easy to crush, due to the arrangement therebetween of the movement regulating members formed by the inner surface of the curving portion of the insertion tube or other contained parts consisting of resin tubes, it is naturally possible to prevent damage of the soft members, such as deformation of the soft members caused when the curving portion is curved and the members hard to crush the soft members, or damage on the covering of the surfaces of the members with soft surfaces caused as a result of the members with hard surfaces and the members with soft surfaces being brought into firm contact with each other when the curving portion is curved. Further, there is no fear of the movement regulating members pressurizing the contained parts to forcibly deform the same, thus preventing or minimizing damage of the contained parts due to pressurization. Further, damage of the surface of the containedparts, etc. can be prevented or minimized.

Further, since there is arranged a forceps channel between two adjacent wire guides of a plurality of wire guides through which wires for curving the curving portion pass, and the movement regulating member is arranged close to the wire guides opposed to those two wire guides, the wire guides are prevented from coming into contact with the contained parts. If the movement regulating member is flawed, the contained parts are not flawed, thus preventing as much as possible the surface of the contained parts, etc. from being flawed by the wire guides.

## Claims

1. An endoscope, comprising:
an insertion tube to be inserted into a body cavity, the insertion tube including a hard distal end portion, a curving portion continuous with the hard distal end portion, and a flexible portion continuous with the curving portion;
a contained part of the insertion tube which is accommodated at least inside the curving portion; and
at least one movement regulating member which extends inside the curving portion and adapted to regulate at least radial movement of the contained part,
one end portion of the position regulating member passing through a rear connecting portion connecting the curving portion and the flexible portion to extend to the flexible portion side,
a section of a portion of the movement regulating member inserted into the flexible portion from the rear connecting portion being smaller than a section of a portion of the movement regulating member within the curving portion.

2. An endoscope according to Claim 1, wherein the movement regulating member exhibits a maximum section at a minimum radius-of-curvature position where a radius of curvature of the curving portion is minimum.

3. An endoscope according to Claim 2, wherein the portion of the movement regulating member within the curving portion has substantially the same section from a front connecting portion connecting the curving portion and the hard distal end portion to each other to at least the minimum radius-of-curvature position.

4. An endoscope according to Claim 2, wherein the movement regulating member has a maximum section at the minimum radius-of-curvature position, and has a section smaller than the maximum section at the front connecting portion and the rear connecting portion.

5. An endoscope according to any one of Claims 1 through 4, wherein another end portion of the movement regulating member is fixed to the hard distal end portion.

6. An endoscope according to any one of Claims 1 through 5, wherein the movement regulating member is tapered such that its section diminishes from a substantially central position of the curving portion toward the one end portion.

7. An endoscope, comprising:
an insertion tube having a hard distal end portion and a flexible portion respectively on both sides of a curving portion that freely curves;
a plurality of contained parts arranged inside the insertion tube; and
at least one movement regulating member regulating radial movements of the contained parts, the movement regulating member being formed by a resin tube whose sectional configuration is capable of elastic deformation with respect to the adjacent contained parts.

8. An endoscope according to Claim 7, wherein
the insertion tube comprises a plurality of wire guides which are fixed to an inner surface thereof and which allow insertion of wires for curving the curving portion,
the contained parts including a forceps channel,
the movement regulating member being arranged at a position in close proximity to outer peripheral portions of the wire guides situated to be opposed to an arrangement position of the forceps channel inside the curving portion, and being provided between the adjacent contained parts and the outer peripheral portions of the wire guides so that the adjacent contained parts may not be brought into direct contact with the outer peripheral portions of the wire guides.

9. An endoscope according to Claim 8, wherein
the plurality of wire guides comprise two wire guides which are adjacent to each other with the forceps channel being arranged therebetween, and wire guides opposing to the two wire guides,
the movement regulating member being arranged in close proximity to the opposing wire guides.

10. An endoscope according to Claim 8 or 9, wherein
the plurality of wire guides are provided at four circumferential positions and comprise two wire guides adjacent to each other and remaining two wire guides opposing to the two wire guides,
the forceps channel being arranged between the two adjacent wire guides,
the movement regulating member being arranged in close proximity to each of the two remaining opposing wire guides.

11. An endoscope according to any one of Claims 7 through 10, wherein the movement regulating member has rigidity toward an outer periphery thereof lower than rigidity toward an outer periphery of the adjacent contained parts.

12. An endoscope according to any one of Claims 7 through 11, wherein the adjacent contained parts include an image pickup data cable and a light guide, and the movement regulating member is a tube made of a resin softer than a covering of the adjacent contained parts.

13. An endoscope according to any one of Claims 7 through 12, wherein the movement regulating member has a maximum diameter larger than a gap formed between the adjacent contained parts or between an inner surface of the curving portion and the adjacent contained parts, and is inserted into this gap in a deformed state.

14. An endoscope, comprising:
an insertion tube having a hard distal end portion and a flexible portion respectively on both sides of a curving portion that freely curves, the insertion tube including a plurality of wire guides which are fixed to an inner surface thereof and which allow insertion of wires for curving the curving portion;
a plurality of contained parts including a forceps channel arranged inside the insertion tube; and
at least one movement regulating member regulating radial movements of the contained parts,
the movement regulating member being arranged at a position in close proximity to outer peripheral portions of the wire guides situated to be opposed to an arrangement position of the forceps channel inside the curving portion, and being provided between the contained parts adjacent to the movement' regulating member and the outer peripheral portions of the wire guides so that the adjacent contained parts may not be brought into direct contact with the outer peripheral portions of the wire guides.

15. An endoscope according to Claim 14, wherein
the plurality of wire guides comprise two wire guides which are adjacent to each other with the forceps channel being arranged therebetween, and wire guides opposing to the two wire guides,
the movement regulating member being arranged in close proximity to the opposing wire guides.

16. An endoscope according to Claim 14 or 15, wherein
the plurality of wire guides are provided at four circumferential positions and comprise two wire guides adjacent to each other and remaining two wire guides opposing to the two wire guides,
the forceps channel being arranged between the two adjacent wire guides,
the movement regulating member being arranged in close proximity to each of the two remaining opposing wire guides.
